# FASCICULE DE BREVET EUROPEEN

(11) **EP 0 625 913 B1**
(45) Date de publication et mention de la délivrance du brevet: **22.07.1998**
(21) Numéro de dépôt: 93904160.4
(22) Date de dépôt: 05.02.1993
(51) Int. Cl.: A61L 9/14, A61L 9/01

(54) **PROCEDE DE DECONTAMINATION ET DE DETOXIFICATION APPLIQUE AU GENIE SANITAIRE DE L'HABITAT**
VERFAHREN ZUR DECONTAMINIERUNG UND ENTGIFTUNG IN DER RAUMHYGIENE
DECONTAMINATING AND DETOXIFYING METHOD FOR DOMESTIC SANITATION

(30) Priorité: 14.02.1992 FR 9201962
(43) Date de publication de la demande: 30.11.1994
(73) Titulaire: BLANC, Michel, F-83310 Port-Grimaud (FR); L'ENTREPRISE COUTHEILLAS SA, 94373 Sucy-en-Brie Cedex (FR)
(72) Inventeur: BLANC, Michel, 83310 Port-Grimaud (FR)
(74) Mandataire: Somnier, Jean-Louis
(86) Numéro de dépôt international: FR9300121
(87) Numéro de publication internationale: WO9315774

(56) Documents cités:
- EP-A- 0 036 339
- EP-A- 0 231 084
- WO-A-90/12600
- AT-B- 391 934
- CLINICAL ALLERGY vol. 5, no. 1, Mars 1975, pages 109 - 114 A. PENAUD ET AL. 'METHODS OF DESTROYING HOUSE DUST PYROGLYPHID MITES' cité dans la demande

## Description

La présente invention a pour objet un procédé de décontamination et de détoxification appliqué au génie sanitaire de l'habitat.

Le secteur technique de l'invention et son application sont dans le domaine de l'hygiène et du génie sanitaire de l'habitat et de la vie domestique.

Les termes écologie et environnement sont devenus des lieux communs surtout en ce qui concerne la nature, mais leur définition doit inclure également l'étude de l'habitat : on peut noter en effet que le mot "écologie" est issu éthymologiquement du mot grec oikos, qui veut dire maison et logos science. De plus cet habitat est contaminé par toutes sortes de particules dites vives : celles-ci sont alors responsables d'une pathologie spécifique, qui a été dénommée "poumon de maison" dans la publication et la conférence effectuées par MM. Michel BLANC et Bruno BLAIVE au "Forum Contaminé Expert" de Versailles les 13, 14 et 15 Septembre 1989; ce terme a été défini comme regroupant les états pathologiques dus aux agresseurs multiples de la pollution intérieure des habitats, provoquée à la fois par les composants de la poussière de maison produite par les animaux, les végétaux, les bactéries, les insectes, les pollens, les moisissures, les virus etc... et qui créent la biocontamination, et par les particules issues de la combustion du tabac et des produits en provenance de l'extérieur dus à la combustion industrielle et à la circulation automobile.

Il y a sommation et interférence entre ces très diverses particules dans le sens d'effets délétères majorés et pathologiques, par exemple ceux des particules de la combustion et de la biocontamination. C'est le cas du tabac et des moisissures, de la nicotine et des acariens.

Il faut noter que la contamination de l'atmosphère intérieure est aussi dépendante de la contamination de l'atmosphère extérieur, qu'elle lui est liée, et qu'elle lui est toujours supérieure.

Cette contamination est différente de la biocontamination, car elle est physique (gaz rares, électromagnétiques etc...) et chimique (combustion industrielle, SO2, NO2 etc...). Elle vient encore interférer sur la configuration des particules de l'air ambiant domestique, que l'être humain inhale à raison de 800 millions de particules par jour, dont la plus grande partie est absorbée par l'organisme.

Leur qualité et leur quantité font que d'une façon habituelle, elles ne sont pas nocives, au contraire parfois même. Mais lorsqu'elles fixent des adjuvants toxiques et/ou contaminés, qu'elles se concentrent en grande quantité (ce qui est le cas dans les habitats confinés et mal ventilés), alors leur toxicité propre apparaît.

Ces particules sont en particulier générées par les déjections des acariens, eux-mêmes élevés dans un milieu de culture composite et mutant.

Les acariens font de la poussière de maison un véritable compost, comme ceux de la forêt font l'humus. Ils trouvent dans l'habitat un biotope toujours propice relevant de nombreux facteurs déjà cités, et en particulier des conditions de température et d'humidité favorables à leur développement, et cela d'autant plus dans les habitats modernes, en particulier dans les salles de bain et les systèmes de ventilation.

Il existe un véritable recyclage permanent par les acariens des particules ainsi générées qui sont alors concentrées par ceux-ci : il est admis que l'allergène majeur du "poumon de maison" est contenu dans les déjections, "chiures", des acariens. Le dosage colorimétrique semi quantitatif de la guanine métabolite azoté est un véritable index de contamination de la poussière de maison, tout en étant spécifique de leur présence. C'est ainsi que ce test est retrouvé positif dans les poussières de menuiserie, de boulangerie, les poussières de poulailler, tout autant que dans la poussière de maison : il existe ainsi une bonne corrélation entre les manifestations cliniques liées à l'allergie des poussières de maison, des acariens et ce test.

Or la menace constante d'une pathologie respiratoire liée à l'inhalation de particules est en progression inquiétante alors que les traitements curatifs n'ont jamais été aussi largement employés et aussi efficaces. Cet échec paradoxal peut s'expliquer par une méconnaissance et une détérioration de l'hygiène élémentaire de l'habitat, et par l'insuffisance des moyens d'assainissement de l'air ambiant, alors que tout laisse à penser que l'origine de la pollution est essentiellement, au départ, extérieure.

Les conditions de vie en habitat réduit, surpeuplé, confiné, la régression évidente de l'hygiène de vie domestique, ont fait croire alors à l'apparition de maladies dites de "civilisation".

En fait, cette pathologie pulmonaire est liée à l'air ambiant de l'habitat.

L'architecture, les économies d'énergie, la pollution liée à la combustion, et notamment le tabagisme passif, la présence des animaux domestiques, la modification des moeurs, les conditions de chauffage productrices de condensations, la présence de salles de bain peu ou pas ventilées, génératrices d'humidité, les systèmes de ventilation etc, toutes ces causes transforment le biotope de l'être vivant qui en dépend et qui en est indissociable.

Ainsi, des études et des recherches se sont développées pour proposer des procédés et des produits, soit d'une part pour le traitement des personnes elles-mêmes afin de combattre et soigner leurs allergies, avec des médicaments adaptés, mais ce n'est pas le cas de la présente invention, soit pour la destruction de la source de ces allergies, qui est la règle fondamentale en allergologie, c'est-à-dire des acariens qui dominent depuis plus de dix ans les débats et les préoccupations des thérapeutes par l'importance des réactions réaginiques qu'il engendre.

On peut citer dans ce cadre la publication de MM. PENAUD, NOURRIT, AUTRAN, TIMON-DAVID, JAQUET FRANCILLON et CHARPIN sur des "methods of destroing house pyroglyphid mites" dans la revue Clinical Allergy de 1975 Volume 5 - pages 109 à 114; et les conférences de MM. BLANC et BOUTIN les 14, 15 et 16 Novembre 1989 à BRUXELLES sur "la destruction des acariens et biodécontamination de l'environnement domestique".

Ces publications et conférences, et l'on pourrait en citer bien d'autres, ont porté essentiellement sur les résultats probants de l'action clinique de produits acaricides utilisés en aérosols d'atmosphère à l'aide d'un "brumisateur"; ces produits, dont un en particulier a été testé avec succès dans ce cadre, et qui était utilisé en application particulière en milieu hospitalier depuis plus de quarante ans dans le domaine de la désinfection des literies, sont à base d'huiles essentielles, et sont du reste connus depuis longtemps, non seulement pour leur effets anti acariens, mais aussi anti fongiques et anti bactériens.

L'action de ces produits est donc prouvée dans ces cas d'applications particulières, en permettant un dépôt des particules de produits sur les surfaces, dans le but d'y détruire essentiellement les acariens : l'idée fait alors utiliser ces produits acaricides électivement sur les matelas par exemple, à l'aide de bombes aérosols, qui ne produisent pas en fait un véritable aérosol, mais un effet de bombage avec des particules comprises entre 20 et 100 µm; cette propulsion par bombe présente des inconvénients majeurs tels que les problèmes liés à l'ozone, leur prix etc..., mais utilisée les fabricants et distributeurs de ces produits.

C'est ainsi que les apports cliniques de la modification de l'environnement particulier de l'asthmatique allergique aux acariens, ont pu être observés et étudiés avec un certain succès.

Cependant malgré ces résultats intéressants et prometteurs, auxquels il faut rajouter dans l'évolution du génie sanitaire de l'habitat, celle des choix de matériaux, des types de ventilation, de l'orientation des pièces, qui permettent aussi de réduire les causes de pollution et de mauvaise hygiène, la pathologie respiratoire liée à l'inhalation des particules de l'habitat, augmente quand même, la nocivité des particules étant concentrée par les acariens, qui eux-mêmes sont la principale cause d'allergie, tel que nous l'avons définie ci-dessus.

Le problème posé est alors de pouvoir assurer une meilleure décontamination de l'habitat qui soit encore plus efficace que les procédés actuels présentés ci-dessus, qui sont orientés sur le traitement des surfaces et la destruction des acariens considérés comme l'allergène responsable principal.

L'inventeur a constaté en effet, à l'encontre des idées reçues et des principes ci-dessus que, dans certains habitats où l'on trouve cependant des acariens en grande quantité, les personnes qui y vivent ne sont pas allergiques à ceux-ci et n'ont aucune indisposition : il a donc constaté qu'il pouvait y avoir des acariens sains et que même ceux-ci sont nécessaires pour l'équilibre de la nature, et que vouloir les détruire n'était sans doute pas la solution absolue, d'autant plus que dans d'autres habitats, où peu d'acariens ont été relevés, des occupants avaient des signes d'allergie.

Il a donc été considéré que plutôt que de traiter des surfaces et les acariens directement, il valait mieux traiter la source de la pollution, que sont alors les poussières elles-mêmes.

Une solution au problème posé est dans le cadre de la présente invention, de préparer chaque pièce dudit habitat pour que tous les éléments mobiliers qui le composent soient exposés à l'air ambiant de ladite pièce; de fermer toutes les ouvertures extérieures de celle-ci; de diffuser ledit produit en aérosol vrai depuis le centre de la pièce en particules de 0,2 à 2 µm maximum pendant une durée suffisante; de traiter ainsi par le brouillard émis et composé par lesdites particules de produit l'ensemble du volume d'air de la pièce considérée et toutes les surfaces exposées; d'arrêter ensuite la diffusion dudit produit, de garder close la pièce considérée pendant une durée suffisante d'action du produit, avant d'aérer ladite pièce; de nettoyer les surfaces par simple essuyage humide.

De préférence, ledit produit utilisé est une formulation riche de dix constituants, et qui contient en particulier des huiles essentielles telles que l'huile essentielle de lilas, l'huile essentielle de citron, l'huile essentielle de citronnelle, des essences aromatiques telles que le terpinéol pour un pourcentage de 20% environ regroupant l'ensemble des huiles essentielles et des essences aromatiques, et des dérivés phénoliques d'origine naturelle, tels que l'acide benzoïque, le salol et le thymol pour un minimum de 4%, et des produits antiseptiques tel le triclosan pour 0,2% environ de la composition totale, l'ensemble de ces composants ci-dessus étant en solution dans un solvant aliphatique également d'origine naturelle pour les 70 à 76% restants.

Dans un mode préférentiel de réalisation on diffuse ledit produit en aérosol pendant une durée déterminée, définie suivant le volume de ladite pièce, et suivant le débit diffusé par le brumisateur, tel que la quantité de produits en suspension dans ledit volume de pièce soit au moins d'un ml/m³, ce qui représente un temps d'opération de 15 minutes environ pour une pièce moyenne. Le temps durant lequel la pièce doit rester ensuite close est de quelques heures en principe, de 3 heures.

Le résultat est un nouveau procédé et une nouvelle application de produits dans le cadre de la décontamination et la détoxification de l'habitat.

Ce procédé suivant l'invention répond en effet au problème posé. car il s'attaque directement aux poussières toxiques avant que les acariens concentrent celles-ci et les rejettent : en effet le procédé suivant l'invention permet, grâce au produit utilisé et à son aérolisation par diffusion gazeuse en fines particules, d'obtenir des aérosols vrais qui font éclater directement les bactéries qui peuvent se situer sur lesdites poussières; on peut également considérer que le produit ainsi diffusé a une action ionisante et précipitante sous cette forme, et se comporte en véritables "anti particules", destructeurs de bactéries, fixé sur une particule porteuse (référence Loi de Henry sur la bactéricidie en phase gazeuse).

On détoxifie ainsi d'une façon efficace l'atmosphère lui-même mais également bien sûr les surfaces qui sont les seules traitées à l'heure actuelle par la mise en oeuvre des produits considérés.

On peut définir à ce propos en complément des explications cidessus ce que signifie le terme "toxique" d'après le dictionnaire : "se dit d'une substance nocive pour un organe vivant"; et la détoxification ou détoxication est la destruction et la diminution de la toxicité par neutralisation du pouvoir toxique de certains corps par leur combinaison avec d'autres substances in vitro et in vivo par l'action de certains organes.

Le procédé suivant l'invention consiste donc en l'utilisation simultanée d'un appareil aéroliseur d'atmosphère générateur de fines particules submicroniques avec un mélange d'huiles essentielles reconnues propres à une action de neutralisation, de décontamination et de détoxification des particules en suspension dans l'air ambiant de l'ensemble d'un habitat contaminé, et également présents sur les surfaces horizontales et verticales de cet habitat.

Selon les références et les publications citées précédemment sur l'analyse des nombreux phénomènes de différentes natures, qui concourent à rendre toxiques les particules de l'air ambiant inhalé, l'inventeur a considéré que si un moyen permet de décontaminer celle-ci, en modifiant leur nature, d'assainir l'air ambiant en faisait baisser leur quantité par agglutination et précipitation, et de détruire les particules biologiques telles que les moisissures génératrices d'endotoxine, ce procédé serait alors le bienvenu; ceci est d'autant plus intéressant dans une situation épidémiologique de plus en plus grave des maladies respiratoires liées à la toxicité des poussières inhalées, bien que cette façon d'aborder le problème, et non pas de traiter celui-ci au niveau des acariens ou du sujet allergique lui-même, a été jusqu'à présent repoussée et non retenue par les milieux professionnels concernés.

Cette approche sur l'aspect de la toxicité de la poussière qui peut être ainsi éliminée grâce au procédé de la présente invention, et donc nouvelle et d'autant plus inventive qu'elle va à contre-courant des principes utilisés à ce jour.

Pour obtenir l'effet voulu du procédé de la présente invention, l'appareil utilisé est un générateur d'un brouillard de fines particules, d'où le nom de brumisateur : c'est un aéroliseur d'atmosphère qui va diffuser dans l'ensemble de l'habitat, pièce par pièce, de la cave au grenier, des aérosols submicroniques du mélange approprié.

Afin d'obtenir la dimension de particules souhaitées de 0,2 à 2 µm maximum, le principe de fonctionnement de cet appareil réside préférentiellement dans l'utilisation e la force centrifuge fournie par un plateau tournant à grande vitesse pour fragmenter un liquide qui a été parallèlement aspiré par un cône de succion.

Les particules émises alors par le générateur sont des aérosols vrais et la finesse des particules obtenues en fait des aérosols secs, qui ne tachent pas les surfaces sur lesquelles ils se déposent et les différencient singulièrement des aérosols émis par les bombes par diffusion, qui ne sont pas des aérosols vrais, mais des particules comprises entre 20 et 100 µm.

On connaît par ailleurs des dispositifs de désinfection de local par générateur d'aérosols, tel que celui décrit dans la demande EP 036339 et pulvérisant dans l'atmosphère un premier microbrouillard d'un dérivé d'aldéïde formique ; le dispositif utilisé pour cela pulvérise la solution désinfectante avec des particules inférieures à 5 µm pour améliorer l'agressivité du produit, mais celui-ci doit alors être ensuite éliminé de l'air ambiant du fait de cette agressivité : pour cela, on fait passer l'atmosphère ainsi traitée dans une enceinte remplie d'un deuxième microbrouillard d'une solution aqueuse neutralisant le dérivé d'aldéïde formique, et on en sépare à la sortie les particules ; ceci constitue un traitement assez complexe, sans doute efficace mais qui de plus utilise un produit, certes désinfectant, mais agressif, même pour l'homme. Son application concerne du reste les salles d'opération et non l'habitat.

Dans la présente invention, le produit utilisé tel que décrit ci-dessus est très différent et de préférence constitué par une formulation originale riche de dix constituants, dont la partie active principale est constituée d'huiles essentielles, qui sont des produits connus du reste depuis au moins 1928, et qui confèrent audit produit des propriétés acaricides, anti fongiques, anti bactériennes, qui sont confirmées par l'ensemble des travaux engagés par les utilisateurs de ce type de produit depuis de nombreuses années.

Ce produit est dénué de toute toxicité pour l'homme et parfaitement toléré par les plantes et les animaux. C'est un produit d'utilisation en présence humaine. Il est par ailleurs ininflammable et ne tache absolument pas lorsqu'il est diffusé sous forme d'aérosols vrais.

Sur le plan physique, ce produit a un pouvoir d'agglutination et de précipitation des particules en suspension dans l'air ambiant et des particules déposées sur les surfaces environnantes. Cette propriété est mise en valeur dans les décontaminations acaricides de l'habitat pour lesquelles on expose au produit choisi, notamment les matelas, car c'est le lieu privilégié où se concentrent les particules générées par les acariens, et les surfaces difficiles d'accès tel que l'intérieur des placards et des penderies par exemple, où nichent également les acariens, les moisissures et les bactéries : c'est cependant uniquement par une action directe des aérosols décontaminants et acaricides sur ces surfaces qu'est utilisé jusqu'à ce jour les produits considérés pour cela.

Le procédé et l'application particulière du produit dans le cadre de la présente invention permet non seulement de conserver ces mêmes qualités de produits indiquées ci-dessus, mais en plus de pouvoir traiter directement la poussière de maison pour la détoxifier à l'origine avant même qu'elle puisse se déposer sur les surfaces et être concentrée ensuite par les acariens; en effet, les particules sont constamment en suspension dans l'air ambiant et sont donc accessibles que par d'autres particules en suspension ayant un grand pouvoir de diffusion, tel que l'assure un brumisateur du type suivant l'invention; les acariens peuvent alors continuer à évoluer dans la maison, sans concentrer de toxicité , et donc sans influence sur les occupants : ils ne seront donc plus la cause d'allergie.

Le procédé suivant l'invention est à répéter à un intervalle de temps suffisant pour maintenir cette qualité de l'hygiène de l'air ambiant à un niveau de non toxicité évitant alors d'avoir à traiter curativement et médicalement les personnes pouvant se situer dans l'habitat.

C'est à ce titre que la présente invention se situe dans le domaine du génie sanitaire de l'habitat et de la vie domestique et ne se situe absolument pas à un niveau médical quelconque, car il n'est pas une thérapeutique appliquée à un malade, mais uniquement un moyen de maintenance de l'habitat à un état de salubrité satisfaisant; Il apporte une mesure d'hygiène, complément indispensable aux consignes thérapeutiques médicales.

## Revendications

1. Procédé de décontamination et de détoxification de l'habitat, utilisant un produit à base d'huiles essentielles et un brumisateur dudit produit, et tel qu'on prépare chaque pièce dudit habitat pour que tous les éléments mobiliers qui le composent soient accessibles et que l'on traite toutes les surfaces exposées par ledit produit et que l'on nettoie ensuite les surfaces par simple essuyage humide, comprenant les étapes suivantes :
- on ferme toutes les ouvertures extérieures de ladite pièce et on expose tous les éléments mobiliers à traiter à l'air ambiant de cette pièce ;
- on diffuse ledit produit en aérosol et on traite ainsi par le brouillard émis et composé par lesdites particules de produit l'ensemble du volume d'air de la pièce considérée ;
- on arrête ensuite la diffusion dudit produit, on maintient la pièce close pendant une durée donnée et on aère ladite pièce, caractérise en ce que :
- l'on diffuse ledit produit en aérosol vrai depuis le centre de la pièce, en particules de 0,2 à 2 µm maximum pendant une durée suffisante et déterminée, définie suivant le volume de ladite pièce, et suivant le débit diffusé par le brumisateur, tel que la quantité de produits en suspension dans ledit volume de pièce soit au moins d'un ml/m³ et que le brouillard ainsi émis décontamine et détoxifie les particules de poussière de l'habitat.

2. Procédé de décontamination et de détoxification de l'habitat suivant la revendication 1, caractérisé en ce que ledit produit utilisé est une formulation riche de dix constituants, et qui contient en particulier des huiles essentielles telles que huile essentielle de lilas, huile essentielle de citron, huile essentielle de citronnelle et des essences aromatiques telles que terpinéol et des dérivés phénoliques d'origine naturelle, tels que l'acide benzoïque, le salol et le thymol et des produits antiseptiques tel que le triclosan, l'ensemble étant en solutions dans un solvant aliphatique également d'origine naturelle.

3. Application de produits à base d'huiles essentielles diffusés dans les pièces d'un habitat pour traiter les surfaces accessibles de celles-ci, caractérisée en ce que ledit produit est utilisé pour désintoxiquer les particules de poussière de l'air ambiant, par action en phase gazeuse, grâce à sa diffusion en aérosol vrai de particules de 0,2 à 2 µm pendant plusieurs heures.

4. Application de produits à base d'huiles essentielles suivant la revendication 3, caractérisée en ce que ledit produit utilisé est composé d'huiles essentielles telles que l'huile essentielle de lilas, l'huile essentielle de citron, l'huile essentielle de citronnelle, des essences aromatiques telles que le terpinéol et des dérivés phénoliques d'origine naturelle, tels que l'acide benzoïque, le salol et le thymol et des produits antiseptiques tels que le triclosan, l'ensemble étant en solutions dans un solvant aliphatique également d'origine naturelle.

5. Application de produits à base d'huiles essentielles suivant l'une quelconque des revendications 3 à 4, caractérisé en ce que ledit produit est diffusé dans l'air ambiant dudit habitat jusqu'à concurrence au moins d'une quantité égale à 1 ml/m³.

## Claims

1. Process for the decontamination and detoxification of a dwelling, using a product based on essential oils and an atomizer for said product, and such that each room of said dwelling is prepared so that all the elements of furniture which compose it are accessible and that all the exposed surfaces are treated with said product and the surfaces are then cleaned by a simple damp wipe, comprising the following steps:
- all the outer openings of said ram are closed and all the elements of furniture to be treated are exposed to the ambient air of this room;
- said product is diffused as an aerosol and all the volume of air of the room in question is thus treated with the mist emitted and composed of said particles of product;
- diffusion of said product is then stopped, the room is maintained closed for a given duration and said room is aired, characterised in that:
- said product is diffused as a true aerosol from the centre of the room in particles of 0.2 to 2 µm maximum for a determined and sufficient period of time, defined as a function of the volume of said room, and as a function of the flowrate diffused by the atomizer, such that the quantity of products in suspension in said volume of room is at least one ml/m³ and that the mist thus emitted decontaminates and detoxifies the particles of dust in the dwelling.

2. Process for decontamination and detoxification of a dwelling according to Claim 1, characterised in that said product used is a rich formulation of ten constituents, and which contains in particular essential oils such as essential oil of lilac, essential oil of lemon, essential oil of citronella and aromatic essences such as terpineol and phenolic derivatives of natural origin, such as benzoic acid, salol and thymol and antiseptic products such as triclosan, all being in solution in an aliphatic solvent likewise of natural origin.

3. Application of products based on essential oils diffused in the rooms of a dwelling to treat the accessible surfaces thereof, characterized in that said product is used for detoxicating the particles of dust in the ambient air, by action in gaseous phase, thanks to the diffusion as true aerosol of particles of 0.2 to 2 µm for several hours.

4. Application of products based on essential oils according to Claim 3, characterised in that said product used is composed of essential oils such as essential oil of lilac, essential oil of lemon, essential oil of citronella, aromatic essences such as terpineol and phenolic derivatives of natural origin, such as benzoic acid, salol and thymol and antiseptic products such as triclosan, all being in solution in an aliphatic solvent likewise of natural origin.

5. Application of products based on essential oils according to any one of Claims 3 to 4, characterised in that the quantity of said product diffused in the ambient air of said dwelling is at least equal to 1 ml/m³.

## Patentansprüche

1. Verfahren zur Dekontaminierung und Entgiftung von Wohnbereichen unter Verwendung eines Produkts auf der Basis von ätherischen Ölen und einer Zerstäubungsvorrichtung für dieses Produkt, wobei man jeden Raum dieses Wohnbereichs so vorbereitet, daß sämtliche darin vorhandenen Einrichtungsgegenstände zugänglich sind, wobei man sämtliche freiliegenden Flächen mit dem Produkt behandelt und anschließend die Flächen durch einfaches feuchtes Wischen reinigt, umfassend die folgenden Stufen:
- man schließt sämtliche nach außen gehende Öffnungen des Raums und setzt sämtliche zu behandelnde Einrichtungsgegenstände der Umgebungsluft dieses Raums aus;
- man verbreitet das Produkt als Aerosol und behandelt das gesamte Luftvolumen des betreffenden Raums mit dem gebildeten Nebel, der aus den Teilchen des Produkts zusammengesetzt ist;
- man beendet anschließend die Verbreitung dieses Produkts, hält den Raum für eine bestimmte Zeitspanne geschlossen und belüftet dann den Raum,
dadurch gekennzeichnet, daß man
- das Produkt als echtes Aerosol von der Raummitte aus in Form von Teilchen von 0,2 bis maximal 2 µm für eine ausreichende Zeitspanne, die entsprechend dem Volumen des Raums und entsprechend dem durch die Zerstäubungsvorrichtung verteilten Ausstoß festgelegt wird, so verteilt, daß die Menge der Produkte, die sich im Raumvolumen in suspendiertem Zustand befinden, mindestens 1 ml/m³ beträgt und daß der auf diese Weise verbreitete Nebel die Staubteilchen des Wohnbereichs dekontaminiert und entgiftet.

2. Verfahren zur Dekontaminierung und Entgiftung von Wohnbereichen nach Anspruch 1, dadurch gekennzeichnet, daß es sich beim verwendeten Produkt um eine zehn Bestandteile enthaltende Zubereitung handelt, die insbesondere ätherische Öle, wie ätherisches Fliederöl, ätherisches Zitronenöl und ätherisches Zitronellenöl, aromatische Essenzen, wie Terpineol, und Phenolderivate natürlichen Ursprungs, wie Benzoesäure, Phenylsalicylat und Thymol, sowie antiseptische Produkte, wie Triclosan, enthält, wobei das gesamte Produkt sich in Lösung in einem aliphatischen Lösungsmittel, das ebenfalls natürlichen Ursprungs ist, befindet.

3. Verwendung von Produkten auf der Basis von ätherischen Ölen, wobei die Produkte in Räumen eines Wohnbereiches zur Behandlung zugänglicher Oberflächen in den Räumen verteilt werden, dadurch gekennzeichnet, daß das Produkt zur Entgiftung von Staubteilchen der Umgebungsluft unter mehrstündiger Einwirkung in der Gasphase aufgrund seiner Verteilung in Form eines echten Aerosols mit Teilchen von 0,2 bis 2 µm verwendet wird.

4. Verwendung von Produkten auf der Basis von ätherischen Ölen nach Anspruch 3, dadurch gekennzeichnet, daß das verwendete Produkt aus ätherischen Ölen, wie ätherisches Fliederöl, ätherisches Zitronenöl und ätherisches Zitronellenöl, aromatischen Essenzen, wie Terpineol, Phenolderivaten natürlichen Ursprungs, wie Benzoesäure, Phenylsalicylat und Thymol, sowie antiseptischen Produkten, wie Triclosan, zusammengesetzt ist, wobei das gesamte Produkt in Form einer Lösung in einem aliphatischen Lösungsmittel, das ebenfalls natürlichen Ursprungs ist, vorliegt.

5. Verwendung von Produkten auf der Basis von ätherischen Ölen nach einem der Ansprüche 3 und 4, dadurch gekennzeichnet, daß das Produkt in der Umgebungsluft des Wohnbereichs bis zum Vorliegen einer Menge von mindestens 1 ml/m³ verteilt wird.
